# EUROPÄISCHE PATENTANMELDUNG

(11) **EP 2 319 490 A2**
(43) Veröffentlichungstag der Anmeldung: **11.05.2011**
(21) Anmeldenummer: 10168390.2
(22) Anmeldetag: 05.07.2010
(51) Int. Cl.: A61K 8/81, A61Q 19/10

(54) **Körperreinigungsmittel mit verbesserter Sensorik**

(30) Priorität: 04.09.2009 DE 102009029218
(71) Anmelder: Henkel AG & Co. KGaA, 40589 Düsseldorf (DE)
(72) Erfinder: Janßen, Frank, 41470, Neuss (DE); Georgelin, Camille, 91270, Vigneux sur Seine (FR)

(57) **Zusammenfassung**

Tensidhaltige Mittel mit angenehmer Produkthaptik und ohne Klebrigkeit sowie mit guter Deposition pflegender Wirkstoffe umfassen in einem wässrigen oder wässrig-alkoholischen Träger - jeweils bezogen auf das Gewicht der anwendungsbereiten Zusammensetzung - (A) 0,01 bis 5 Gew.-% mindestens eines ersten Copolymers, ausgewählt aus Copolymeren von (a1) mindestens einem sauren Vinylmonomer, ausgewählt aus Acrylsäure und Methacrylsäure oder einem Salz davon, (a2) mindestens einem hydrophoben nichtionischen Vinylmonomer, ausgewählt aus Acrylsäureestern und Methacrylsäureestern, (a3) mindestens einem ersten assoziativen Monomer und (a4) mindestens einem Monomer, das ausgewählt ist aus der Gruppe bestehend aus einem zweiten assoziativen Monomer, das sich von dem ersten assoziativen Monomer unterscheidet, einem semihydrophoben Monomer und einer Kombination davon; wobei die assoziativen Monomere (i) eine ethylenisch ungesättigte Endgruppe, (ii) einen hydrophilen Mittelabschnitt und (iii) eine hydrophobe oder semihydrophobe Endgruppe aufweisen; Sowie (B) 0,01 bis 2,5 Gew.-% mindestens eines zweiten Homo- und/oder Copolymers, ausgewählt aus ganz oder teilweise neutralisierten Homo- und/oder Copolymeren der Acrylsäure und/oder Methacrylsäure, und (C) 1 bis 35 Gew.-% eines oder mehrerer Tenside.

## Beschreibung

Die Erfindung betrifft tensidhaltige Reinigungsmittel auf der Basis einer speziellen Wirkstoffkombination sowie die Verwendung dieser Wirkstoffkombination zur Verbesserung des Hautgefühls dieser Reinigungsmittel.

Reinigungsmittel für die Haut, wie sie beispielsweise als flüssige Seifen, Duschbäder, Schaumbäder, Dusch- und Waschgele im Handel erhältlich sind, müssen nicht nur ein gutes Reinigungsvermögen aufweisen, sondern sollen weiterhin für die Haut und die Schleimhäute gut verträglich sein und auch bei häufiger Anwendung nicht zu starker Entfettung oder Hauttrockenheit führen. Daneben erwartet der Verbraucher eine ansprechende Produkthaptik. Die Produkte sollen als cremig und reichhaltig empfunden werden, weshalb eine bestimmte Viskosität gewünscht wird. Die üblicherweise zur Erzielung höherer Viskositäten eingesetzten Verdicker haben allerdings oft den Nachteil, daß sie den Produkten eine gewisse vom Verbraucher als unangenehm empfundene Klebrigkeit verleihen. Darüber hinaus verhindern die üblichen Verdicker oft die Penetration von pflegenden Wirkstoffen aus dem Reinigungsmittel in die Haut.

Der vorliegenden Erfindung lag die Aufgabe zugrunde, tensidhaltige Reinigungsmittel bereitzustellen, die eine hohe Viskosität und damit eine angenehme Produkthaptik aufweisen, ohne daß die Produkte deshalb als unangenehm klebrig empfunden werden. Neben der Verbesserung des Hautgefühls bestand eine weitere Aufgabe der Erfindung darin, tensidhaltige Reinigungsmittel bereitzustellen, die die Deposition pflegender Wirkstoffe aus der abzuspülenden Reinigungsmittelmatrix ermöglichen.

Es wurde nun gefunden, daß eine bestimmte Polymerkombination in Mitteln mit einem bestimmten Tensidgehalt eine angenehme Produkthaptik herbeiführen kann, ohne daß die Produkte klebrig werden und ohne daß die Deposition pflegender Wirkstoffe behindert wird.

Ein erster Gegenstand der vorliegenden Erfindung ist eins kosmetische oder dermatologisch-topische Zusammensetzung, umfassend in einem wässrigen oder wässrig-alkoholischen Träger - jeweils bezogen auf das Gewicht der anwendungsbereiten Zusammensetzung -
• (A) 0,01 bis 5 Gew.-% mindestens eines ersten Copolymers, ausgewählt aus Copolymeren von
   o (a1) mindestens einem sauren Vinylmonomer, ausgewählt aus Acrylsäure und Methacrylsäure oder einem Salz davon,
   o (a2) mindestens einem hydrophoben nichtionischen Vinylmonomer, ausgewählt aus Acrylsäureestern und Methacrylsäureestern,
   o (a3) mindestens einem ersten assoziativen Monomer und
   o (a4) mindestens einem Monomer, das ausgewählt ist aus der Gruppe bestehend aus einem zweiten assoziativen Monomer, das sich von dem ersten assoziativen Monomer unterscheidet, einem semihydrophoben Monomer und einer Kombination davon;
   wobei die assoziativen Monomere (i) eine ethylenisch ungesättigte Endgruppe, (ii) einen hydrophilen Mittelabschnitt und (iii) eine hydrophobe oder semihydrophobe Endgruppe aufweisen;
• (B) 0,01 bis 2,5 Gew.-% mindestens eines zweiten Homo- und/oder Copolymers, ausgewählt aus ganz oder teilweise neutralisierten Homo- und/oder Copolymeren der Acrylsäure und/oder Methacrylsäure,
• (C) 1 bis 35 Gew.-% eines oder mehrerer Tenside.

Die erfindungsgemäßen Zusammensetzungen enthalten mindestens ein Tensid und eine Gesamtmenge an Tensiden von 1 bis 35 Gew.-%, jeweils bezogen auf das gesamte Mittel.

Ganz besonders bevorzugte erfindungsgemäße Zusammensetzungen enthalten - bezogen auf ihr Gewicht - 2 bis 32,5 Gew.-%, vorzugsweise 5 bis 30 Gew.-%, weiter bevorzugt 7,5 bis 27,5 Gew.-% und insbesondere 10 bis 25 Gew.-% Tensid(e) aus den Gruppen der anionischen und/oder nichtionischen und/oder amphoteren und/oder zwitterionischen Tenside.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate,
- sulfatierte Fettsäurealkylenglykolester,
- Monoglyceridsulfate und Monoglyceridethersulfate.

Bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Besonders bevorzugte anionische Tenside sind die Alkali- oder Ammoniumsalze des Laurylethersulfates mit einem Ethoxylierungsgrad von 2 bis 4 EO.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈- C₂₄- Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ -Acylsarcosin.

Die erfindungsgemäßen Mittel enthalten vorzugsweise- bezogen auf das Gewicht des anwendungsbereiten Mittels - 0,1 bis 10 Gew.-% mindestens eines Betains der Formel (I) 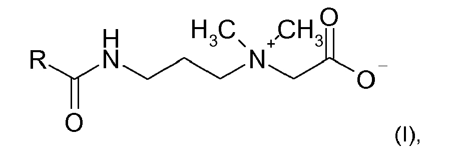 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten bevorzugt sind und als Cocoamidopropylbetaine bezeichnet werden. Besonders bevorzugt werden erfindungsgemäß Tenside der Formel (I) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₉-C(O)-NH-(CH₂)₃N⁺(CH₃)₂CH₂COO⁻
H₃C-(CH₂)₁₁-C(O)-NH-(CH₂)₃N⁺(CH₃)₂CH₂COO⁻
H₃C-(CH₂)₁₃-C(O)-NH-(CH₂)₃N⁺(CH₃)₂CH₂COO⁻
H₃C-(CH₂)₁₅-C(O)-NH-(CH₂)₃N⁺(CH₃)₂CH₂COO⁻
H₃C-(CH₂)₇-CH=CH-(CH₂)₇-C(O)-NH-(CH₂)₃N⁺(CH₃)₂CH₂COO⁻

Besonders bevorzugt werden Tenside der Formel (I) innerhalb engerer Mengenbereiche eingesetzt. Hier sind erfindungsgemäße Mittel bevorzugt, die - bezogen auf ihr Gewicht - 0,25 bis 7,5 Gew.-%, weiter bevorzugt 0,5 bis 6 Gew.-% und insbesondere 1 bis 4 Gew.-% Tensid(e) der Formel (I) enthalten.

Die erfindungsgemäßen Mittel enthalten vorzugsweise - bezogen auf das Gewicht des anwendungsbereiten Mittels - 0,1 bis 10 Gew.-% mindestens eines Betains der Formel (II) 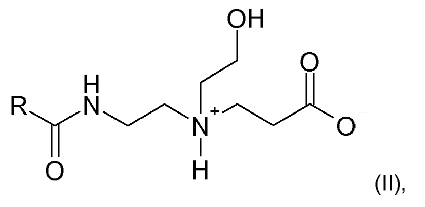 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amphoacetate bezeichnet, wobei die Vertreter, die sich von Kokosfettsäuren ableiten bevorzugt sind und als Cocoamphoactetate bezeichnet werden.

Aus herstellungstechnischen Gründen enthalten Tenside dieses Typs immer auch Betaine der Formel (III) 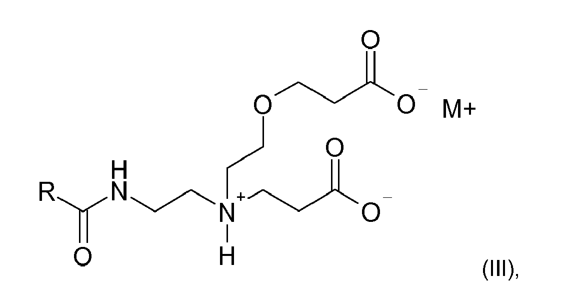 in der R für einen geradkettigen oder verzweigten, gesättigten oder ein- bzw. mehrfach ungesättigten Alkyl- oder Alkenlyrest mit 8 bis 24 Kohlenstoffatomen und M für ein Kation steht. Diese Tenside werden nach der INCI-Nomenklatur als Amphodiacetate bezeichnet, wobei die Vertreter, die sich von Kokosfettsäuren ableiten bevorzugt sind und als Cocoamphodiactetate bezeichnet werden.

Besonders bevorzugt werden erfindungsgemäß Tenside der Formel (II) eingesetzt, die ein Gemisch der folgenden Vertreter sind:
H₃C-(CH₂)₇-C(O)-NH-(CH₂)₂NH⁺(CH₂CH₂OH)CH₂CH₂COO⁻
H₃C-(CH₂)₉-C(O)-NH-(CH₂)₂NH⁺(CH₂CH₂OH)CH₂CH₂COO⁻
H₃C-(CH₂)₁₁-C(O)-NH-(CH₂)₂NH⁺(CH₂CH₂OH)CH₂CH₂COO⁻
H₃C-(CH₂)₁₃-C(O)-NH-(CH₂)₂NH⁺(CH₂CH₂OH)CH₂CH₂COO⁻
H₃C-(CH₂)₁₅-C(O)-NH-(CH₂)₂NH⁺(CH₂CH₂OH)CH₂CH₂COO⁻
H₃C-(CH₂)₇-CH=CH-(CH₂)₇-C(O)-NH-(CH₂)₂NH⁺(CH₂CH₂OH)CH₂CH₂COO⁻

Besonders bevorzugt werden Tenside der Formel (II) innerhalb engerer Mengenbereiche eingesetzt. Hier sind erfindungsgemäße Mittel bevorzugt, die - bezogen auf ihr Gewicht - 0,25 bis 7,5 Gew.-%, weiter bevorzugt 0,5 bis 6 Gew.-% und insbesondere 1 bis 4 Gew.-% Tensid(e) der Formel (II) enthalten.

Ganz besonders bevorzugte erfindungsgemäße Zusammensetzungen sind **dadurch gekennzeichnet, daß** sie - bezogen auf ihr Gewicht - 0,1 bis 10 Gew.-% .-%, vorzugsweise 0,25 bis 7,5 Gew.-%, weiter bevorzugt 0,5 bis 6 Gew.-% und insbesondere 1,0 bis 4 Gew.-% mindestens eines Betains der Formel (I) 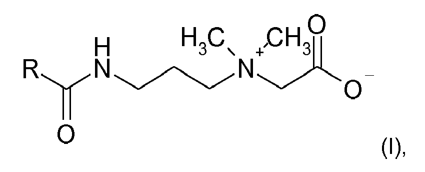 in der R für einen geradkettigen oder verzweigten, gesättigten oder ein- bzw. mehrfach ungesättigten Alkyl- oder Alkenlyrest mit 8 bis 24 Kohlenstoffatomen steht, und/oder 0,1 bis 10 Gew.-% .-%, vorzugsweise 0,25 bis 7,5 Gew.-%, weiter bevorzugt 0,5 bis 6 Gew.-% und insbesondere 1,0 bis 4 Gew.-% mindestens eines Betains der Formel (II) 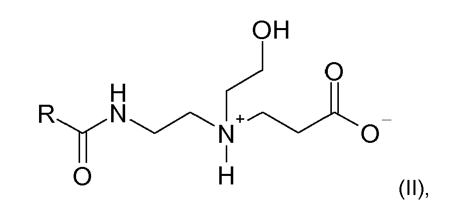 in der R für einen geradkettigen oder verzweigten, gesättigten oder ein- bzw. mehrfach ungesättigten Alkyl- oder Alkenlyrest mit 8 bis 24 Kohlenstoffatomen steht, enthalten. Zusammenfassend sind erfindungsgemäße Zusammensetzungen bevorzugt, bei denen der Rest R in den Formeln (I) und (II) ausgewählt ist aus H₃C-(CH₂)₇-, H₃C-(CH₂)₉-, H₃C-(CH₂)₁₁-, H₃C-(CH₂)₁₃-, H₃C-(CH₂)₁₅-, H₃C-(CH₂)₇-CH=CH-(CH₂)₇-, oder Mischungen aus diesen.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-C₆- Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl, beispielsweise Rizinusöl-hydriert+40 EO,
- Polyolfettsäureester,
- alkoxilierte Triglyceride,
- alkoxilierte Fettsäurealkylester,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen
besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Ganz besonders bevorzugte erfindungsgemäße Zusammensetzungen sind **dadurch gekennzeichnet, daß** sie - bezogen auf ihr Gewicht - 0,1 bis 10 Gew.-% .-%, vorzugsweise 0,5 bis 9,5 Gew.-%, weiter bevorzugt 1 bis 9 Gew.-% und insbesondere 2,5 bis 8 Gew.-% mindestens eines Alkylpolyglycosids enthalten.

Als bevorzugte nichtionische Tenside haben sich auch die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Gemäß einer weiteren Ausführungsform der Erfindung können die tensidhaltigen Reinigungsmittel weiterhin kationische Tenside vom Typ der quartären Ammoniumverbindungen, der Esterquats und der Amidoamine enthalten.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Als wesentliches weiteres Element enthalten die erfindungsgemäßen Zusammensetzungen ein Verdickersystem aus zwei Polymeren (A) und (B).

Das erste Polymer ist ein Copolymer aus (a1) mindestens einem sauren Vinylmonomer, ausgewählt aus Acrylsäure und Methacrylsäure oder einem Salz davon, (a2) mindestens einem hydrophoben nichtionischen Vinylmonomer, ausgewählt aus Acrylsäureestern und Methacrylsäureestern, (a3) mindestens einem ersten assoziativen Monomer und (a4) mindestens einem Monomer, das ausgewählt ist aus der Gruppe bestehend aus einem zweiten assoziativen Monomer, das sich von dem ersten assoziativen Monomer unterscheidet, einem semihydrophoben Monomer und einer Kombination davon;
wobei die assoziativen Monomere (i) eine ethylenisch ungesättigte Endgruppe, (ii) einen hydrophilen Mittelabschnitt und (iii) eine hydrophobe oder semihydrophobe Endgruppe aufweisen;

Das saure Vinylmonomer, ausgewählt aus Acrylsäure und Methacrylsäure oder einem Salz davon, macht vorzugsweise 10 bis 75% Gew.-%, besonders bevrozugt 25 bis 65 Gew.-% und insbesondere 30 bis 60 Gew.-% der gesamten Monomermischung aus.

Das nichtionische Vinylmonomer macht vorzugsweise 10 bis 90% Gew.-%, weiter bevorzugt 25 bis 75 Gew.-% und insbesondere 30 bis 60 Gew.-% der gesamten Monomermischung aus. Im Rahmen der vorliegenden Erfindung besonders geeignete nichtionische Vinylmonomere sind Acrylsäure- und Methacrylsäure-Ester, wie beispielsweise C₁-C₁₈ Alkylester von Acrylsäure und Methacrylsäure, Methacrylamidoethyl-N-ethylen-harnstoff und Mischungen daraus. Besonders bevrozugte nichtionische Vinylmonomere sind Acrylsäure-C1- bis C4-Alkylester und Methacrylsäure-C1- bisC4-Alkylester wie Ethylacrylat oder Methylmethacrylat.

Besonders bevorzugte erfindungsgemäße Zusammensetzungen sind **dadurch gekennzeichnet, daß** die nichtionischen Vinylmonomere (a2) ausgewählt sind aus Acrylsäure-C₁₋₄-alkylestern und Methacrylsäure- C₁₋₄-alkylestern und wobei das Monomer (a4) ein assoziatives Monomer ist, das sich von (a3) in den hydrophoben Endgruppen unterscheidet, die unabhängig voneinander aus C₈-bis C₄₀-Kohlenwasserstoffendgruppen ausgewählt sind.

Ganz besonders bevorzugte erfindungsgemäße Zusammensetzungen sind **dadurch gekennzeichnet, da**ß die Monomermischung als hydrophobes nichtionisches Vinylmonomer (a2) PEG-25 C10-30 Alkylether Methacrylat umfaßt.

Vorzugsweise machen die assoziativen Monomere in der Monomermischung unabhängig voneinander jeweils 0,1 bis 25 Gew.-%, besonders bevorzugt 0,25 bis 20 Gew.-% und insbesondere 0,5 bis 15 Gew.-% aus. Erfindungsgemäß geeignete assoziative Monomere besitzen eine ethylenisch ungesättigte Gruppe (i) zur Polymerisation mit den anderen Monomeren des Systems, eine Polyoxyalkylen-Mittelsektion (ii), um selektive hydrophile Eigenschaften des Polymers sicherzustellen und eine hydrophobe Endgruppe (iii), um selektive hydropobe Eigenschaften des Polymers sicherzustellen. Die Gruppe (i) stammt vorzugsweise von einer alpha-beta-ethylenisch ungesättigten Mono- oder Dicarbonsäure oder deren Anhydrid, besonders bevorzugt von einer C₃ oder C₄ Mono- oder Dicarbonsäure oder ihrem Anhydrid. Alternativ kann die Gruppe (i) des assoziativen Monomers von einem Allylether oder Vinylether, einem nichtionischen vinyl-substituierten Urethanmonomer oder einem vinylsubstituierten Harsntoffreaktionsprodukt abgeleitet sein. Die Mittelsektion (ii) ist vorzugsweise ein Polyoxyalkylensegment von 5 bis 250, vorzugsweise 10 bis 120 und insbesondere 15 bis 60 wiederkehrenden C₂-C₇-Alkylenoxideinheiten. Bevorzugte Gruppen (ii) sind Polyoxyethylen-, Polyoxypropylen- und Polyoxybutylen-Segmente, die 5 bis 150, weiter bevorzugt 10 bis 100 und inssbesondere 15 bis 60 Ethylen-, Propylen- oder Butylenoxideinheiten ausweisen, wobei die Alkylenoxideinheiten im Block oder willkürlich verteilt sein können.

Die hydrophobe Endgruppe (iii) des assoziativen Monomers ist vorzugsweise eine Kohlenwasserstoffgruppe aus den folgenden Gruppen: lineare C₈-C₄₀ Alkylgruppen, aryl-substituierte C₂-C₄₀ Alkylgruppen, C₂-C₄₀ alkyl-substituierte Phenylgruppen, verzweigte C₈-C₄₀ Alkylgruppen, C₈-C₄₀ Cycloalkylgruppen; komplexe C₈-C₈₀ Ester. Beispiele für solche Gruppen (iii) sind Capryl (C₈), Iso-octyl (verzweigtes C₈), Decyl (C₁₀), Lauryl (C₁₂), Myristyl (C₁₄), Cetyl (C₁₆), Cetearyl (C₁₆-C₁₈), Stearyl (C₁₈), Isostearyl (verzweigtes C₁₈), Arachidyl (C₂₀), Behenyl (C₂₂), Lignoceryl (C₂₄), Cerotyl (C₂₆), Montanyl (C₂₈), Melissyl (C₃₀), Lacceryl (C₃₂). Solche linearen oder verzweiten Alkylgruppen mit 8 bis 40 Kohlenstoffatomen können aus natürlichen Quellen gewonnen warden, zum Beispiel Alkylgruppen aus hydriertem Erdnußöl, Sojaöl und Canolaöl (überwiegend C₁₈), hydriertem Talgfett (C₁₆-C₁₈), hydriertenm C₁₀-C₃₀-Terpenolen wie hydriertem Geraniol (verzweigtes C₁₀), hydriertem Farnesol (verzweigtes C₁₅), hydriertem Phytol (verzweigtes C₂₀). Beispiele für geeignete C₂-C₄₀ alkylsubstituierte Phenylgruppen sind Octylphenyl, Nonylphenyl, Decylphenyl, Dodecylphenyl, Hexadecylphenyl, Octadecylphenyl, Isooctylphenyl, sec-Butylphenyl, usw.. Geeignete cyclische C₈-C₄₀ Kohlenswasserstoffe sind beispielsweise Gruppen aus tierischen Quellen wie Cholesterol, Lanosterol, 7-Dhydrocholesterol, usw.; aus pflanzlichen Quellen wie Phytosterol, Stigmasterol, Campesterol, usw. und aus Pilzen und Hefen wie beispielsweise Ergosterol, Mycosterol, usw.. Andere carbocyclische Gruppen sind beispielsweise Cyclooctyl, Cyclododecyl, Adamantyl, Decahydronaphthyl und Gruppen, die aus natürlichen Carbocyclen gewoonnen warden, z.B. Pnen, hydriertem Retinol, Campher, Isobornylalkohol, usw.. Beispiele für aryl-substituierte C₂-C₄₀ Alkylgruppen sind Styryl (2-Phenylethyl), Distyryl (2,4-Diphenylbutyl), Tristyryl (2,4,6-Triphenylhexyl), 4-Phenylbutyl, 2-;ethyl-2-phenylethyl, Tristyrylphenolyl, usw.. beispiele für geeignete komplexe C₈-C₈₀ Esters sind hydriertes Castor Oil (überwiegend das Triglycerid von 12-Hydroxystearinsäure); 1,2-Diacylglycerine wie 1,2-Distearylglycerin, 1,2-Dipalmitylglycerin, 1,2-Dimyristylglycerin usw., Di-, Tri-, oder Polyester von Zuckern wie 3,4,6-Tristearylglucose, 2,3-Dilaurylfructose usw. und Sorbitanester.

Besonders bevorzugte assoziative Monomere sind polyethoxyliertes Cetylmethacrylat (CEM), polyethoxyliertes Cetearylmethacrylat (CSEM), polyethoxyliertes Stearyl(meth)acrylat, polyethoxyliertes Arachidyl(meth)acrylat, polyethoxyliertes Behenylmethacrylat (BEM), polyethoxyliertes Cerotyl(meth)acrylat, polyethoxyliertes Montanyl(meth)acrylat, polyethoxyliertes Melissyl(meth)acrylat, polyethoxyliertes Lacceryl(meth)acrylat, polyethoxyliertes Tristyrylphenolmethacrylate(TEM), polyethoxyliertes hydriertes Castor Oil Methacrylat (HCOEM), polyethoxyliertes Canola(meth)acrylat und polyethoxyliertes Cholesterinmethacrylat (CHEM), wobei die polyethoxylierte Sequenz jeweils 5 bis 100, vorzugsweise 10 bis 80 und insbesondere 15 bis 60 EO-Einheiten umfaßt.

Wenn des Copolymer zwei verschiedene assoziative Monomere umfasst, werden die beiden hydrophoben Endgruppen der beiden assoziativen Monomere unabhängig voneinander aus den gleichen oder aus verschiedenen Gruppen der vorstehend genannten Kohlenwasserstoffe ausgewählt. Falls die Endgruppen der beiden assoziativen Monomere aus der gleichen Gruppe der vorstehend genannten Kohlenwasserstoffe ausgewählt werden, ist es bevorzugt, daß sie sich um mindestens 8 C-Atome voneinander unterscheiden.

Die semihydrophoben Monomere umfassen vorzugsweise 0,1 bis 25 Gew.-%, besonders bevorzugt 0,5 bis 20 Gew.-% und insbesondere 1 bis 15 Gew.-% der gesamten Monomermischung. Erfindungsgemäß geeignete semihydrophobe Monomere besitzen zwei Teile: (i) eine polymerisierbare ethylenisch ungesättigte Endgruppe zur Reaktion mit den anderen Monomeren der monomermischung und (ii) eine Polyoxyalkylengruppierung als nicht-hydrophobe Endgruppe. Die ungesättigte Endgruppe (i) leitet sich vorzugsweise von einem Allylether, einem Vinylether, einem nichtionischem Urethanmonomer, einer alpha-beta-ungesättigten Mono- oder Dicarbonsäure oder einem Anhydrid davon, vorzugsweise einer C₃ oder C₄ Mono- oder Dicarbonsäure oder einem Anhydrid davon. Die polymerisierbare ethylenisch ungesättigte Endgruppe (i) kann auch von einer ungesättigten C₈-C₃₀ Fettsäuregruppierung mit mindestens einer freien Carboxyfunktion abgeleitet sein. Eine solche C₈-C₃₀-Gruppe ist Bestandteil der ungesättigten Endgruppe (i) und unterscheidet sich von den hydrophoben Gruppen der assoziativen Monomere, die von der ungesättigten Endgruppe des assoziativen Monomers durch hydrophilie "spacer"-Gruppen getrennt ist. Die Polyoxyalkylen-Gruppe (ii) enthält ein langkettiges Polyoxyalkylensegment, welches im Wesentlichen gleich ist mit der hydrophilen Gruppe der assoziativen Monomere.

Bevorzugte Gruppen (ii) sind Polyoxyethylen-, Polyoxypropylen- und Polyoxybutylen-Segmente, die 2 bis 250, weiter bevorzugt 10 bis 100 Ethylen-, Propylen- oder Butylenoxideinheiten ausweisen, wobei die Alkylenoxideinheiten im Block oder willkürlich verteilt sein können

Bevorzugte semihydrophone Monomere sind solche der folgenden Formeln: R¹CH=CR¹-A-(CH₂)ₚ-(O)ᵣ-(R³O)ᵥ-R⁴
oder
D-A-(CH₂)ₚ-(O)ᵣ(R³O)ᵥ-R⁴
wobei in jeder Formel jedes R¹ unabhängig voneinander für H, C₁-C₃₀ alkyl, -C(O)OH, oder -C(O)OR² steht; R² steht für C₁-C₃₀ Alkyl; A bedeutet -CH₂C(O)O- , -C(O)O-, -O-, -CH₂O-, -NHC(O)NH-, -C(O)NH-. -Ar-(CE₂)_{z} NHC(O)O-, -Ar-(CE₂)_{z}-NHC(O)NH-, oder -CH₂CH₂NHC(O)-; Ar ist ein divalenter Arylrest; E ist H oder Methyl; z ist 0 or 1; p ist eine Zahl zwischen 0 und 30, und r steht für 0 oder 1, mit der Maßgabe, daß wenn p 0 ist, auch r 0 ist , und wenn p eine Zahl von 1 bis 30 ist, r für 1 steht; (R³O)ᵥ ist eine Polyoxyalkylengruppierung, welche ein Homopolymer, ein statistisch verteiltes Copolymer oder ein Blockcopolymer von C₂-C₄ Oxyalkyleneinheiten, wobei R³ für C₂H₄, C₃H₆, C₄H₈ steht und v eine Zahl von 5 bis 250, vorzugsweise von 5 bis 100, weiter bevorzugt von 10 bis 80, und insbesondere von 15 bis 60 bedeutet; R⁴ ist H or C₁-C₄ Alkyl; und D ist eine ungesättigte C₈-C₃₀ Alkyl- oder eine ungesättigte carboxy-substituierte C₈-C₃₀ Alkylgruppe.

Besonders bevorzugte semihydrophobe Monomere enthalten Monomere der folgenden Formeln: CH₂=CH-O-(CH₂)ₐ-O-(C₃H₆O)_{b}-(C₂H₄O)_{c}-H
oder
CH₂=CH-CH₂-O-(C₃H₆O)_{d}-(C₂H₄O)ₑ-H;
worin a 2, 3, oder 4 ist; b eine Zahl von 1 bis 10, vorzugsweise von 2 bis 8, besonders bevorzugt von 3 bis 7 ist; c für eine Zahl von 5 bis 50, vorzugsweise von 8 bis 40 und insbesondere von 10 bis 30 steht; d eine Zahl von 1 bis 10, vorzugsweise von 2 bis 8, besonders bevorzugt von 3 bis 7 ist; und e für eine Zahl von 5 bis 50, vorzugsweise von 8 bis 40 steht.

Beispiele bevorzugter semihydrophober Monomere sind kommerziell erhältliche polymerisierbare Emulgatoren wie EMULSOGEN® R109, R208, R307, RAL109, RAL208, and RAL307 (Clariant Corporation); BX-AA-E5P5 (Bimax, Inc.); und MAXEMUL® 5010 und 5011 (Uniqema) sowie Mischungen davon. Besonders bevorzugte semihydrophobe Monomere sind EMULSOGEN® R109, R208, und R307, BX-AA-E5P5, MAXEMUL® 5010 und 5011, sowie Mischungen davon

Laut Herstellerangaben ist EMULSOGEN® R109 ein statistisch ethoxyliert/propoxylierter 1,4-Butanediol-Vinylether der Formel CH₂=CH-O(CH₂)₄O(C₃H₆O)₄(C₂H₄O)₁₀H; EMULSOGEN® R208 ist ein statistisch ethoxyliert/propoxylierter 1,4-Butanediol-Vinylether der Formel CH₂=CH-O(CH₂)₄O(C₃H₆O)₄-(C₂H₄O)₂₀H; EMULSOGEN® R307 ist ein statistisch ethoxyliert/propoxylierter 1,4-Butanediol-Vinylether der Formel CH₂=CH-O(CH₂)₄O(C₃H₆O)₄-(C₂H₄O)₃₀H; EMULSOGEN® RAL109 ist ein statistisch ethoxyliert/propoxylierter Allylether der Formel CH₂=CHCH₂O-(C₃H₆O)₄(C₂H₄O)₁₀H; EMULSOGEN® RAL208 ist ein statistisch ethoxyliert/propoxylierter Allylether der Formel CH₂CHCH₂O(C₃H₆O)₄(C₂H₄O)₂₀H; EMULSOGEN® RAL307 ist ein statistisch ethoxyliert/propoxylierter Allylether der Formel CH₂CHCH₂O(C₃H₆O)₄(C₂H₄O)₃₀H; MAXEMUL® 5010 ist ein carboxy-funktionelles C₁₂-C₁₅ Alkenyl, ethoxyliert mit ca. 24 EO; MAXEMUL® 5011 ist ein carboxy-funktionelles C₁₂-C₁₅ Alkenyl, ethoxyliert mit ca. 34 EO; and BX-AA-E5P5 ist ein statistisch ethoxyliert/propoxylierter Allyether der Formel CH₂=CHCH₂O(C₃H₆O)₅(C₂H₄O)₅H.

Besonders bevorzugte erfindungsgemäße Zusammensetzungen sind **dadurch gekennzeichnet, da**ß die Monomermischung mindestens ein semihydrophobes Monomer (a4) umfasst, das ausgewählt ist aus Monomeren mit einer der folgenden Formeln R¹CH=CR¹-A-(CH₂)ₚ-(O)ᵣ-(R³O)ᵥ-R⁴
oder
D-A-(CH₂)ₚ-(O)ᵣ(R³O)ᵥ-R⁴
wobei in jeder der Formeln jedes R¹ unabhängig H, C₁-C₃₀-Alkyl, -C(O)OH oder -C(O)OR² ist; R² C₁-C₃₀-Alkyl ist; A -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- oder -CH₂CH₂NHC(O)- ist; Ar ein zweiwertiges Aryl ist; E H oder Methyl ist; z 0 oder 1 ist; p eine ganze Zahl im Bereich von 0 bis etwa 30 ist und r 0 oder 1 ist, mit der Maßgabe, dass, wenn p 0 ist, r 0 ist, und wenn p im Bereich von 1 bis etwa 30 liegt, r 1 ist; (R³O)ᵥ ein Polyoxyalkylen ist, welches ein Homopolymer, ein statistisches Copolymer oder ein Blockcopolymer von C₂-C₄-Oxyalkyleneinheiten ist, worin R³ C₂H₄, C₃H₆, C₄H₈ ist und v eine ganze Zahl im Bereich von etwa 5 bis etwa 250 ist, R⁴ H oder C₁-C₄-Alkyl ist; und D ein ungesättigtes C₈-C₃₀-Alkyl oder ein carboxysubstituiertes ungesättigtes C₈-C₃₀-Alkyl ist.

Ganz besonders bevorzugte erfindungsgemäße Zusammensetzungen sind **dadurch gekennzeichnet, da**ß die Monomermischung mindestens ein semihydrophobes Monomer (a4) umfasst, das ausgewählt ist aus Monomeren mit einer der folgenden Formeln CH₂=CH-O-(CH₂)ₐ-O-(C₃H₆O)_{b}-(C₂H₄O)_{c}-H
oder
CH₂=CH-CH₂-O-(C₃H₆O)_{d}-(C₂H₄O)ₑ-H;
worin a 2, 3 oder 4 ist; b eine ganze Zahl im Bereich von 1 bis etwa 10 ist; c eine ganze Zahl im Bereich von etwa 5 bis etwa 50 ist; d eine ganze Zahl im Bereich von 1 bis etwa 10 ist; und e eine ganze Zahl im Bereich von etwa 5 bis etwa 50 ist.

Insbesondere bevorzugte erfindungsgemäße Zusammensetzungen sind **dadurch gekennzeichnet, da**ß die Monomermischung als semihydrophobes Monomer (a4) PEG/PPG-5/5 Allyl Ether umfaßt. Die Copolymere (A), die im rahmen der vorliegenden Erfindung eingesetzt werden, können optional eine weitere Verbindung enthalten, die ausgewählt ist aus der Gruppe bestehend aus Vernetzungsmitteln, Kettenübertragungsmitteln und einer Kombination davon.

Bevorzugte Vernetzungsmittel sind Acrylat- und Methacrylatester von Polyolen, die mindestens zwei Acrylat- und Methacrylatgruppen enthalten, wie beispielsweise Timethylolpropantriacrylat, Trimethylolpropandimethacrylat, Polyethylenglycoldimethacrylat, ethoxyliertes Bsphenol A Dimethacrylat usw.. Bevorzugte Kettenübertragungsmittel sind vorzugsweise ausgewählt aus einer breiten Vielfalt von Thio- und Disulfid-haltigen Verbindungen wie C₁-C₁₈ Alkylmercaptanen, Mercaptocarbonsäuren, Mercaptocarbonsäureestern, Thioestern, C₁-C₁₈ Alkyldisulfiden, Aryldisulfiden, polyfunktionellen Thiolen usw.; Phosphiten und Hypophosphiten; Haloalkylverbindungen wie Tetrachlorkohlenstoff, Bomtrichloromethan usw. und ungesättigten Kettenübertragungsmitteln wie alpha-Methylstyrol.

Zusammenfassend sind erfindungsgemäße Zusammensetzungen bevorzugt, bei denen das erste Copolymer (A) aus mindestens einer zusätzlichen Verbindung hergestellt ist, die ausgewählt ist aus der Gruppe bestehend aus Vernetzungsmitteln, Kettenübertragungsmitteln und einer Kombination davon.

Beorzugte Copolymere, die als Copolymer (A) im rahmen der vorliegenden Erfindung geeignet sind, sind z.B. in US 2003/0207988 A1 beschrieben. Ein besonders bevorzugtes als Copolymer einsetzbares Copolymer hat die INCI-Bezeichnung Polyacrylate-14 und wird unter der Handelsbezeichnung FIXATE™ PLUS von Noveon, Inc. vertrieben.

Unabhängig davon, welche(s) Copolymer(e) (A) in den erfindungsgemäßen Zusammensetzungen enthalten ist/sind, sind erfindungsgemäße Zusammensetzungen bevorzugt, die - bezogen auf ihr Gewicht - 0,02 bis 4 Gew.-%, vorzugsweise 0,05 bis 3 Gew.-%, weiter bevorzugt 0,1 bis 2,5 Gew.-%, noch weiter bevorzugt 0,25 bis 1,5 Gew.-% und insbesondere 0,5 bis 0,95 Gew.-% Copolymer(e) (A) enthalten.

Die erfindungsgemäßen Zusammensetzungen enthalten als Copolymer (B) bezogen auf ihr Gewicht 0,01 bis 2,5 Gew.-% mindestens eines zweiten Homo- und/oder Copolymers, ausgewählt aus ganz oder teilweise neutralisierten Homo- und/oder Copolymeren der Acrylsäure und/oder Methacrylsäure, also ein Homo- oder Copolymer, das mindestens eine Struktureinheit gemäß Formel (B-I) enthält 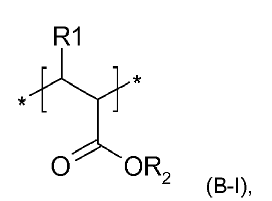 in der R1 für -H oder -CH₃ und R2 für -H oder ein einfach positiv geladenes Metallion oder eine entsprechende Gruppierung oder den n-ten Teil eines n-fach positiv gealdenen Metallions oder einer entsprechenden Gruppierung steht.

Unabhängig von der Wahl der Reste R1 und R2 und gegebenenfalls weiter vorhandenen Mnomerbausteinen in den Copolymeren sind erfindungsgemäße Zusammensetzungen bevorzugt, die als zweites Homo- und/oder Copolymer, ausgewählt aus ganz oder teilweise neutralisierten Homo- und/oder Copolymeren der Acrylsäure und/oder Methacrylsäure, ganz oder teilweise neutralisierte Homo- und/oder Copolymere der Acrylsäure mit Molmassen von 200 bis 10.000 kDa, vorzugsweise von 250 bis 7500 kDa und insbesondere von 500 bis 5000 kDa enthalten.

Die Säurefunktion der Acrylsäure kann vollständig oder nur teilweise neutralisiert vorliegen, wobei der Neutralisationsgrad vom pH-Wert der Mittel abhängt. Vollständig nicht neutralisierte Polyacrylsäuren bzw. Polymethacrylsäuren sind in der Regel nur unterhalb von pH-Werten zwischen 2,5 und 3 beständig, d.h. bei physiologisch verträglichen pH-Werten liegt immer ein Teil der Carboxylgruppen als Carboxylatgruppe vor. Erfindungsgemäß bevorzugte Zusammensetzungen sind **dadurch gekennzeichnet, da**ß der Neutralisationsgrad der ganz oder teilweise neutralisierten Homo- und/oder Copolymere der Acrylsäure und/oder Methacrylsäure 40 bis 100%, vorzugsweise 50 bis 100%, besonders bevorzugt 60 bis 99,5% und insbesondere 70 bis 99% beträgt. Bezogen auf den pH-Wert der Mittel sind erfindungsgemäße Zusammensetzungen bevorzugt, die pH-Werte von 4 bis 8, bevorzugt von 4,5 bis 7,7, besonders bevorzugt von 5 bis 7,5 und insbesondere von 5,5 bis 7,2 aufweisen.

Eine Gruppe von Polymeren, die bevorzugt in den erfindungsgemäßen Zusammensetzungen eingesetzt werden, sind Hompolymere der Acrylsäure, die ganz oder teilweise neutralisiert sind, z.B. mit Ammonium, Natrium, Kalium, Calcium oder Mischungen aus diesen Kationen. Diese Homopolymeren werden vorzugsweise alleine, d.h. nicht in Mischung mit weiteren Copolymeren der Acrylsäure eingesetzt. Andere Polymere, die keine Acrylsäure-Monomere enthalten, können selbstverständlich zugegen sein. Erfindungsgemäß besonders bevorzugte Zusammensetzungen sind demnach **dadurch gekennzeichnet, da**ß sie als Copolymer (B) ausschließlich Homopolymere ganz oder teilweise neutralisierter Polyacrylsäuren, vorzugsweise aus der Gruppe der Homopolymeren mit den INCI-Bezeichnungen Ammonium Polyacrylate und/oder Calcium Potassium Carbomer und/oder Carbomer und/oder Polyacrylic Acid und/oder Potassium Carbomer und/oder Potassium Polyacrylate und/oder Sodium Carbomer und/oder Sodium Polyacrylate enthalten.

Es ist auch möglich und bevorzugt, Copolymere der Acrylsäure erfindungsgemäß zu verwenden. Hier sind erfindungsgemäße Zusammensetzungen besonders bevorzugt, die als zweites Homo-und/oder Copolymer, ausgewählt aus ganz oder teilweise neutralisierten Homo- und/oder Copolymeren der Acrylsäure und/oder Methacrylsäure ein oder mehrere Copolymer(e) aus der Gruppe der Polymere mit den INCI-Bezeichnungen Acrylamide/Sodium Acrylate Copolymer und/oder Acrylates/Aminoacrylates/C10-30 Alkyl PEG-20 Itaconate Copolymer und/oder Acrylates/C10-30 Alkyl Acrylate Crosspolymer und/oder Acrylates/Stearyl Methacrylate Copolymer und/oder Acrylates/Vinyl Isodecanoate Crosspolymer und/oder Allyl Methacrylates Crosspolymer und/oder Ammonium Polyacrylate und/oder Calcium Potassium Carbomer und/oder Corn Starch/Acrylamide/Sodium Acrylate Copolymer und/oder Potassium Carbomer und/oder Potassium Polyacrylate und/oder Sodium Acrylate/Acryloyldimethyl Taurate Copolymer und/oder Sodium Acrylate/Acryloyldimethyltaurate/Dimethylacrylamide Crosspolymer und/oder Sodium Acrylate/Acryloyldimethyltaurate/Acrylamide Colymer und/oder Sodium Acrylates/Vinyl Isodecanoate Crosspolymer und/oder Sodium Acrylate/Vinyl Alcohol Copolymer und/oder Sodium Polyacrylate Starch und/oder Sodium Polymethacrylate enthalten.

Unabhängig davon, welche(s) Copolymer(e) (B) in den erfindungsgemäßen Mittel enthalten ist, sind erfindungsgemäße Zusammensetzungen bevorzugt, die - bezogen auf ihr Gewicht - 0,02 bis 2,0 Gew.-%, vorzugsweise 0,05 bis 1,5 Gew.-%, weiter bevorzugt 0,1 bis 1,25 Gew.-%, noch weiter bevorzugt 0,25 bis 1,0 Gew.-% und insbesondere 0,5 bis 0,95 Gew.-% ganz oder teilweise neutralisierte Homo- und/oder Copolymere der Acrylsäure und/oder Methacrylsäure enthalten. Neben den drei zwingenden Inhaltsstoffen können die erfindungsgemäßen Mittel weitere Inhaltsstoffe enthalten.

Erfindungsgemäß bevorzugte Zusammensetzungen sind **dadurch gekennzeichnet, da**ß sie zusätzlich mindestens einen Pflege-Enhancer aus der Gruppe L-Carnitin und/oder seiner Salze; Panthenol und/oder Panthothensäure; der 2-Furanone und/oder deren Derivate, insbesondere Pantolacton; Taurin und/oder seiner Salze; Niacinamid; Ubichinon Ectoin; Allantoin; Extrakten von Echinacea enthalten.

Diese Pflege-Enhancer werden nachstehend beschrieben

L-Carnitin (IUPAC-Name(R)-(3-Carboxy-2-hydroxypropyl)- N,N,N-trimethylammoniumhydroxid), ist eine natürlich vorkommende, vitaminähnliche Substanz. Es spielt eine essentielle Rolle im Energiestoffwechsel menschlicher, tierischer und pflanzlicher Zellen. L-Carnitin kann über verschiedene Wege im industriellen Maßstab gewonnen werden. Beispielsweise über einen, die körpereigene Biosynthese imitierenden, biotechnologischen Prozess: In großen Fermentationsbehältern wird dabei die Vorstufe von L-Carnitin (y-Butyrobetain) mit Hilfe von gramnegativen Bakterien (Rhizobien) in L-Carnitin umgesetzt.

Als Betain kann L-Carnitin Additionsverbindungen und Doppelsalze bilden. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich.

Erfindungsgemäße bevorzugte Zusammensetzungen sind **dadurch gekennzeichnet, da**ß sie - bezogen auf ihr Gewicht -0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Panthenol (IUPAC-Name: (+)-(R)-2,4-Dihydroxy-N-(3-hydroxypropyl)-3,3-dimethylbutyramid) wird im Körper zu Pantothensäure umgewandelt. Pantothensäure ist ein Vitamin aus der Gruppe der B-Vitamine (Vitamin B5).

Erfindungsgemäße bevorzugte Zusammensetzungen sind **dadurch gekennzeichnet, da**ß sie - bezogen auf ihr Gewicht -0,01 bis 5 Gew.-%, vorzugsweise 0,05 bis 2,5 Gew.-%, besonders bevorzugt 0,1 bis 1,5 Gew.-% und insbesondere 0,25 bis 1 Gew.-% Panthenol ((±)-2,4-Dihydroxy-N-(3-hydroxypropyl)-3,3-dimethyl-butyramid) enthalten.

Erfindungsgemäß bevorzugte Zusammensetzungen enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% mindestens eines 2-Furanonderivats der Formel (Fur-I) und/oder der Formel (Fur-II) 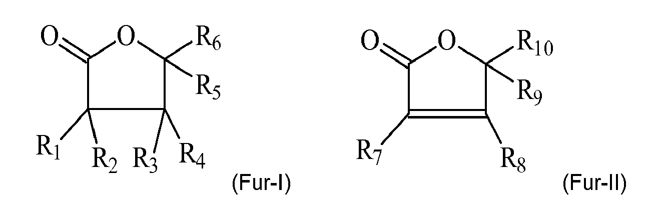 in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht. In einer besonders bevorzugten Ausführungsform der erfindungsgemäßen Lehre wird als Verbindung entsprechend der Formel (Fur-I) (R)-(-)-4-Hydroxymethyl-Υ-butyrolacton und/oder D,L-4-Hydroxymethyl-γ-butyrolacton und/oder (S)-(+)-4-Hydroxymethyl-γ-butyrolacton und/oder R-(-)-2-Hydroxy-3,3-dimethyl-γ-butyrolacton und/oder D,L-2-Hydroxy-3,3-dimethyl-γ-butyrolacton und/oder S(+)-2-Hydroxy-3,3-dimethyl-γ-butyrolacton und/oder 4-Hydroxy-2,5-dimethyl-3(2H)-furanon und/oder Tetrahydro-5-oxo-2-furancarbonsäure und/oder Tetrahydro-5-oxo-2-furancarbonsäure, Na-Salz und/oder Tetrahydro-5-oxo-2-furancarbonsäure, K-Salz und/oder 2,5-Dihydro-5-methoxy-2-furanon und/oder Dihydro-3-hydroxy-4,4-dimethyl-2(3H)-furanon eingesetzt. In einer ganz besonders bevorzugten Ausführungsform der erfindungsgemäßen Lehre wird als Verbindung entsprechend der Formel (Fur-I) Dihydro-3-hydroxy-4,4-dimethyl-2(3H)-furanon eingesetzt.

Ein weiterer, bevorzugter einsetzbarer Pflege-Enhancer, der aktivierende Eigenschaften besitzt, ist das Taurin. Erfindungsgemäß bevorzugte Zusammensetzungen enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflege-Enhancerm in den erfindungsgemäßen Mitteln sind Vitamine, Provitamine oder Vitaminvorstufen. Diese werden nachfolgend beschrieben:
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Unter dem Begriff "Vitamin F" werden üblicherweise essentielle Fettsäuren, insbesondere Linolsäure, Linolensäure und Arachidonsäure, verstanden.

Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Zusammenfassend sind erfindungsgemäße Zusammensetzungen bevorzugt, die zusätzlich - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthält, die vorzugsweise den Gruppen A, B, C, E, F und H zugeordnet werden, wobei bevorzugte Mittel Panthenol ((±)-2,4-Dihydroxy-*N-*(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß bestimmte Chinone eine besondere Eignung als Pflege-Enhancer besitzen. Als weiteren Pflege-Enhancer können die erfindungsgemäßen Mittel daher 0,0001 bis 5 Gew.-% mindestens eines Biochinons der Formel (Ubi) 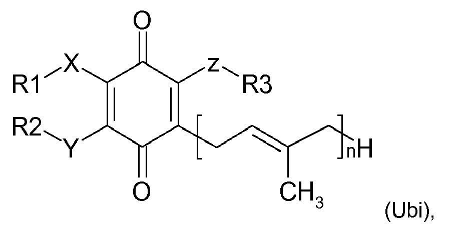 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)- Alkylengruppe, oder einen (C₁-C₆)-Acylrest, wobei bevorzugte Reste unabhängig voneinander ausgewählt sind aus -H, - CH₃, -CH₂CH₃, -(CH₂)₂CH₂, -CH(CH₃)₂, -(CH₂)₃CH₃, -CH(CH₃)CH₂CH₃, - CH₂CH(CH₃)₂, -C(CH₃)₃
- R⁴: steht für -CH₃, -CH₂CH₃, -(CH₂)₂CH₂, -CH(CH₃)₂, - (CH₂)₃CH₃, -CH(CH₃)CH₂CH₃, -CH₂CH(CH₃)₂, -C(CH₃)₃
- n: steht für Werte von 1 bis 20, vorzugsweise von 2 bis 15 und insbesondere für 5, 6, 7, 8, 9, 10.

Besonders bevorzugte erfindungsgemäße Zusammensetzungen sind **dadurch gekennzeichnet, da**ß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 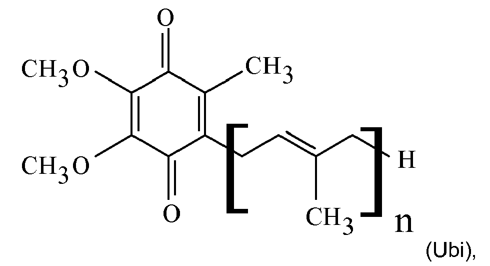 in der n für die Werte = 6, 7, 8, 9 oder 10, besonders bevorzugt für 10 (Coenzym Q10) steht.

Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel **dadurch gekennzeichnet, da**ß sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-b) enthalten 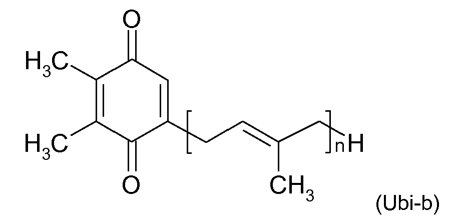 in der n für Werte von 1 bis 20, vorzugsweise von 2 bis 15 und insbesondere für 5, 6, 7, 8, 9, 10 steht, wobei besonders bevorzugt Mittel Plastochinon PQ-9 der Formel 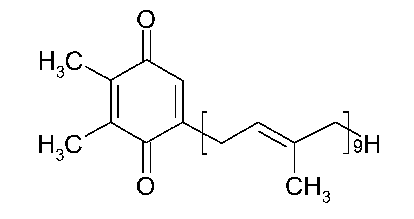 enthalten.

Als weiteren Pflege-Enhacer können die erfindungsgemäßen Mittel Ectoin enthalten. Erfindungsgemäß bevorzugte Zusammensetzungen sind **dadurch gekennzeichnet, da**ß sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Ein weiterer Pflege-Enhancer ist Allantoin. Erfindungsgemäße besonders bevorzugte Zusammensetzungen enthalten - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% 5-Ureidohydantoin (Allantoin).

Als weiterer Pflege-Enhancer eignen sich erfindungsgemäß Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Insbesondere bevorzugt sind Extrakte von Echinacea- oder Moringa-Pflanzen, wobei Echinacea-Extrakte besonders bevorzugt sind. Erfindungsgemäß besonders bevorzugte Zusammensetzungen sind **dadurch gekennzeichnet, da**ß sie - bezogen auf ihr Gewicht - 0,001 bis 10 Gew.-%, insbesondere 0,01 bis 2 Gew.-% Wirkstoff, erhältlich aus Pflanzen der Gattung Echinacea, vorzugsweise aus Presssäften und Extrakten, die aus Echinacea purpurea gewonnen werden, enthalten.

Zur Verbesserung der Elastizität und Festigung der inneren Struktur der mit erfindungsgemäßen Mitteln behandelter Haare können die erfindungsgemäßen Mittel Purin und/oder Purinderivate als Pflege-Enhancer enthalten. Insbesondere die Kombination von Purin und/oder Purinderivaten mit Ubichinonen und/oder Plastochinonen als Pflege-Enhancer führt dazu, daß die mit entsprechenden Mitteln behandelten Haare unter anderem höhere Meßwerte bei der Differenzthermoanalyse und verbesserte Naß- und Trockenkämmbarkeiten zeigen.

Bevorzugte erfindungsgemäße Mittel enthalten Purin und/oder Purinderivate in engeren Mengenbereichen. Hier sind erfindungsgemäß bevorzugte kosmetische Mittel **dadurch gekennzeichnet, da**ß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten. Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher **dadurch gekennzeichnet, da**ß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflege-Enhancer können die erfindungsgemäßen Mittel auch Flavonoide enthalten. Die Flavonoide sind eine Gruppe von wasserlöslichen Pflanzenfarbstoffen und spielen eine wichtige Rolle im Stoffwechsel vieler Pflanzen. Sie gehören zusammen mit den Phenolsäuren zu den Polyphenolen. Es sind weit über 6500 unterschiedliche Flavonoide bekannt, die sich in Flavonole, Flavone, Flavanone, Isoflavonoide und Anthocyane einteilen lassen.

Erfindungsgemäß können Flavonoide aus allen sechs Gruppen eingesetzt werden, wobei bestimmte Vertreter aus den einzelnen Gruppen als Pflege-Enhancer wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Erfindungsgemäß besonders bevorzugte Zusammensetzungen sind **dadurch gekennzeichnet, da**ß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-*O*-rutinosid (Rutin), enthalten.

Bevorzugt ist auch der Einsatz von Bisabolol und/oder Bisabololoxiden als Pflege-Enhancer in den erfindungsgemäßen Mitteln. Hier sind erfindungsgemäße Zusammensetzungen bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Auch Creatin eignet sich erfindungsgemäß als Pflege-Enhancer. Besonders bevorzugte erfindungsgemäße Zusammensetzungen enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% N-Methyl-guanidino-essigsäure (Creatin).

Als weiteren Inhaltsstoff können die erfindungsgemäßen Mittel mit besonderem Vorzug eine oder mehrere Aminosäuren enthalten. Bevorzugte erfindungsgemäße Mittel enthalten eine oder mehrere Aminosäuren in engeren Mengenbereichen. Hier sind erfindungsgemäß bevorzugte Haarreinigungsmittel **dadurch gekennzeichnet, da**ß sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel **dadurch gekennzeichnet, da**ß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus
- Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose
- Disacchariden, insbesondere Saccharose und/oder Maltose und/oder Lactose und/oder Trehalose und/oder Cellobiose und/oder Gentiobiose und/oder Isomaltose.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.

Weiter sind erfindungsgemäß bevorzugte kosmetische Mittel **dadurch gekennzeichnet, da**ß sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat und 0,1 bis 0,25 Gew.-% Glycin,
- 0,005 bis 0,015 Gew.-% Coffein und 0,75 bis 1,5 Gew.-% Saccharose und 0,1 bis 0,25 Gew.-% Glycin,
- 0,005 bis 0,015 Gew.-% Coffein und 0,75 bis 1,5 Gew.-% Fructose und 0,1 bis 0,25 Gew.-% Glycin,
- 0,005 bis 0,015 Gew.-% Coffein und 0,75 bis 1,5 Gew.-% Glucosemonohydrat und 0,1 bis 0,25 Gew.-% Alanin,
- 0,005 bis 0,015 Gew.-% Coffein und 0,75 bis 1,5 Gew.-% Saccharose und 0,1 bis 0,25 Gew.-% Alanin,
- 0,005 bis 0,015 Gew.-% Coffein und 0,75 bis 1,5 Gew.-% Fructose und 0,1 bis 0,25 Gew.-% Alanin,
- 0,005 bis 0,015 Gew.-% Coffein und 0,75 bis 1,5 Gew.-% Glucosemonohydrat und 0,1 bis 0,25 Gew.-% Valin,
- 0,005 bis 0,015 Gew.-% Coffein und 0,75 bis 1,5 Gew.-% Saccharose und 0,1 bis 0,25 Gew.-% Valin,
- 0,005 bis 0,015 Gew.-% Coffein und 0,75 bis 1,5 Gew.-% Fructose und 0,1 bis 0,25 Gew.-% Valin.

Erfindungsgemäße bevorzugte Mittel sind **dadurch gekennzeichnet, da**ß sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.

Erfindungsgemäß besonders bevorzugte Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

Bevorzugte Silikone werden nachstehend beschrieben.

Besonders bevorzugte erfindungsgemäße Mittel sind **dadurch gekennzeichnet, da**ß sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht.

Diese Silikone werden nach der INCI-Nomenklatur als DIMETHICONE bezeichnet.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind. Besonders bevorzugte erfindungsgemäße Mittel enthalten ein oder mehrere aminofunktionelle Silicone. Bevorzugte erfindungsgemäße Mittel sind **dadurch gekennzeichnet, da**ß sie ein aminofunktionelles Silikon der Formel (Si-II)

R'ₐG₃₋ₐ-Si(OSiG₂)ₙ-(OSiG_{b}R'_{2-b})ₘ-O-SiG₃₋ₐ-R'ₐ (Si-II),

enthalten, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, - CH₂CH₂CH₂CH₃, -O-CH₂CH(CH₃)₂, -CH₂CH(CH₃)₂, -O-CH(CH₃)CH₂CH₃, - CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   o -Q-N(R")-CH₂-CH₂-N(R")₂
   o -Q-N(R")₂
   o -Q-N⁺(R")₃A⁻
   o -Q-N⁺H(R")₂A⁻
   o -Q-N⁺H₂(R")A⁻
   o -Q-N(R")-CH₂-CH₂-N⁺R"H₂A⁻,
wobei jedes Q für eine chemische Bindung, -CH₂-, -CH₂-CH₂-, -CH₂CH₂CH₂- , -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind **dadurch gekennzeichnet, da**ß sie mindestens ein aminofunktionelles Silikon der Formel (Si-IIa) 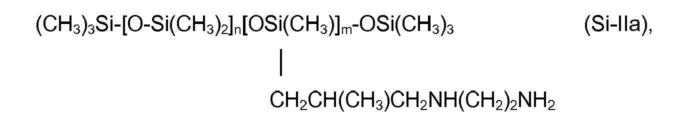 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet. Besonders bevorzugt sind auch erfindungsgemäße Mittel, die ein aminofunktionelles Silikon der Formel (Si-IIb) 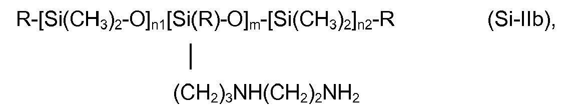 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Erfindungsgemäß bevorzugte Mittel sind **dadurch gekennzeichnet, dass** sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Mit Vorzug sind die Silikone wasserlöslich. Erfindungsgemäß bevorzugte Mittel sind **dadurch gekennzeichnet, da**ß sie mindestens ein wasserlösliches Silikon enthalten.

Weiterhin kann in einer bevorzugten Ausführungsform der Erfindung ein erfindungsgemäßes Mittel auch UV - Filter (I) enthalten. Es eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Zusätzlich kann es sich als vorteilhaft erweisen, wenn in den erfindungsgemäßen Mitteln Penetrationshilfsstoffe und/ oder Quellmittel (M) enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Bevorzugte erfindungsgemäße Zusammensetzungen enthalten - bezogen auf ihr Gewicht - 0,0001 bis 5 Gew.-% Duftstoff(e). Als Duftstoffkomponente können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl. Besonders bevorzugte erfindungsgemäße Zusammensetzungen sind **dadurch gekennzeichnet, da**ß mindestens eine Duftstoffkomponente in einer Gesamtmenge von 0,0001 bis 4 Gew.-%, bevorzugt 0,5 - 2 Gew.-%, jeweils bezogen auf die Gesamtzusammensetzung, enthalten ist.

Als ein weiteres besonders geeignetes Antioxidans können die erfindungsgemäßen Mittel mindestens einen Stoff aus der Gruppe der Xanthophylle, insbesondere Astaxanthin, enthalten. Xanthophylle ist der Gruppenname für Hydroxy-, Epoxy- und Oxo-Derivate von Carotinoiden. Beispiele für erfindungsgemäß geeignete Verbindungen sind (z. B. Canthaxanthin, Citranaxanthin, Flavoxanthin, Rhodoxanthin, Rubixanthin, Zeaxanthin, Lutein, Cryptoxanthin, Violaxanthin und insbesondere Astaxanthin). Die natürlich vorkommenden Xanthophylle. sind meist *all-trans-*Verbindungen.

Erfindungsgemäß bevorzugte Zusammensetzungen enthalten - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 1 Gew.-% Xanthophylle, vorzugsweise Canthaxanthin, Citranaxanthin, Flavoxanthin, Rhodoxanthin, Rubixanthin, Zeaxanthin und ganz besonders bevorzugt Astaxanthin. Als optionalen Inhaltsstoff können die erfindungsgemäßen Mittel zusätzlich mindestens ein DNA-Reparaturenzym enthalten. Als DNA-Reparaturenzyme haben sich im Rahmen der vorliegenden Erfindung insbesondere Photolyase, T4 Endonuclease V und 8-Oxoguanin-glycosylase bewährt. Bevorzugte erfindungsgemäße Zusammensetzungen sind daher **dadurch gekennzeichnet, da**ß sie zusätzlich mindestens ein DNA-Reparaturenzym, vorzugsweise Photolyase und/oder T4 Endonuclease V und/oder 8-Oxoguanin-glycosylase und/oder deren Mischungen enthalten.

Ganz besonders bevorzugte erfindungsgemäße Zusammensetzungen sind **dadurch gekennzeichnet, da**ß das DNA-Reparaturenzym ausgewählt ist aus Photolyase und T 4 Endonuclease V sowie Mischungen dieser Enzyme.

Liposomenverkapselte Photolyase ist im Handel z. B. unter der Produktbezeichnung Photosomes™ (INCI-Bezeichnung: Aqua, Lecithin, Plankton Extract) liposomenverkapselte T4N5 z. B. unter der Bezeichnung Ultrasomes™ (INCI-Bezeichnung: Aqua, Lecithin, Micrococcus Lysate) von der Firma AGI Dermatics, USA, erhältlich. Da sich bei längerer Lagerung sowohl des Handelsproduktes als auch der erfindungsgemäßen Zusammensetzungen zwischen der verkapselten wässrigen, das Enzym enthaltenden Phase und der nicht-verkapselten, äußeren wässrigen Phase ein Gleichgewicht einstellt, liegt ein Teil der DNA-Reparaturenzyme unverkapselt vor. In den erfindungsgemäßen Zusammensetzungen sind die Handelsprodukte Photosomes™ oder Ultrasomes™ bevorzugt in Mengen von 0,1 - 10 Gew.-%, besonders bevorzugt 0,5 - 5,0 Gew.-% und außerordentlich bevorzugt 1,0 - 4,0 Gew.-%, bezogen auf das gesamte Mittel, enthalten. Bevorzugte erfindungsgemäße Zusammensetzungen sind **dadurch gekennzeichnet, da**ß mindestens ein DNA-Reparaturenzym in Gesamtmengen von 0,00001 - 1 Gew.-%, bevorzugt 0,0001 - 0,1 Gew.-%, besonders bevorzugt 0,001 - 0,01 - 0,05 Gew.-% und außerordentlich bevorzugt 0,002 - 0,005 Gew.-%, enthalten ist.

Ganz besonders bevorzugte erfindungsgemäße Zusammensetzungen sind **dadurch gekennzeichnet, da**ß sie das/die DNA-Reparaturenzym(e) - bezogen auf das Gewicht des Hautbehandlungsmittels - in Mengen von 0,00001 - 1 Gew.-%, bevorzugt 0,0001 - 0,1 Gew.-%, besonders bevorzugt 0,001 -0,05 Gew.-% und außerordentlich bevorzugt 0,002 - 0,005 Gew.-%, enthalten.

Ein weiterer Gegenstand der vorliegenden Erfindung ist die Verwendung von Mischungen aus
• (A) mindestens einem ersten Copolymer, ausgewählt aus Copolymeren von
   o (a1) mindestens einem sauren Vinylmonomer, ausgewählt aus Acrylsäure und Methacrylsäure oder einem Salz davon,
   o (a2) mindestens einem hydrophoben nichtionischen Vinylmonomer, ausgewählt aus Acrylsäureestern und Methacrylsäureestern,
   o (a3) mindestens einem ersten assoziativen Monomer und
   o (a4) mindestens einem Monomer, das ausgewählt ist aus der Gruppe bestehend aus einem zweiten assoziativen Monomer, das sich von dem ersten assoziativen Monomer unterscheidet, einem semihydrophoben Monomer und einer Kombination davon; wobei die assoziativen Monomere (i) eine ethylenisch ungesättigte Endgruppe, (ii) einen hydrophilen Mittelabschnitt und (iii) eine hydrophobe oder semihydrophobe Endgruppe aufweisen;
• (B) mindestens einem zweiten Homo- und/oder Copolymer, ausgewählt aus ganz oder teilweise neutralisierten Homo- und/oder Copolymeren der Acrylsäure und/oder Methacrylsäure,
• (C) mindestens eines Tensids
zur Verbesserung des Hautgefühls kosmetischer oder dermatologisch-topischer Zusammensetzungen.

Bezüglich bevorzugter Ausführungsforen der erfindungsgemäßen Verwendung gilt mutatis mutandis das zu den erfindungsgemäßen Mitteln bzw. Zusammensetzungen Gesagte.

Die folgenden Beispiele erläutern den Gegenstand der Erfindung näher.

### Beispiele:

Es wurden folgende Zusammensetzungen hergestellt (alle Angaben in Gew.-%)

| | **V1** | **E** | **V2** |
|---|---|---|---|
| Carbopol ETD 2020 | 0,8 | 0,8 | - |
| Sorbit 70% LS DAB | 3 | 3 | 3 |
| Texapon SB 3 unkonserviert | 5 | 5 | 5 |
| Kokosamidopropylbetain 40% | 7,5 | 7,5 | 7,5 |
| Plantacare 2000 UP | 7 | 7 | 7 |
| LAMESOFT PO 65 | 4 | 4 | 4 |
| Coconut glycerides + 7.3 EO | 0,5 | 0,5 | 0,5 |
| Polymer JR 400 | 0,3 | 0,3 | 0,3 |
| AMP Ultra PC 1000 | 0,8 | 0,8 | 0,8 |
| Triethanolamin 99 % rein | 1,19 | 1,19 | 1,19 |
| Na-benzoat | 0,4 | 0,4 | 0,4 |
| EDTA Natriumsalz, Pulver | 0,1 | 0,1 | 0,1 |
| Natriumhydroxid Perlen | 0,453 | 0,602 | 0,602 |
| Fixate Plus | - | 2,5 | 2,5 |
| Wasser, vollentsalzt | ad 100 | ad 100 | ad 100 |

- Carbopol^{®} ETD 2020: vernetztes Acrylsäurecopolymer, weißes Pulver (INCI-Bezeichnung: Acrylates / C10-30 Alkylacrylate Crosspolymer) (Noveon)
- Texapon^{®} SB3: Laurylsulfosuccinat, Dinatriumsalz (ca. 40% Aktivsubstanz; INCI- Bezeichnung: Disodium Laureth Sulfosuccinate) (Cognis)
- Plantacare^{®} 2000 UP: C₈₋₁₆ Alkylglucosid (ca. 51-55% Aktivsubstanzgehalt in Wasser; INCI- Bezeichnung: Decyl Glucoside, Aqua (Water)) (Cognis)
- Lamesoft^{®} PO65: Alkylpolyglucosid Ölsäuremonoglycerid Gemisch (ca. 65-70% Festkörper; INCI-Bezeichnung: Coco-Glucoside, Glyceryl Oleate, Aqua (Water)) (Cognis)
- Polymer JR^{®} 400: quaternierte Hydroxyethylcellulose (INCI-Bezeichnung: Polyquaternium-10) (Amerchol)
- AMP Ultra PC 2000: 2-Amino-2-methyl-propanol (ca. 94,5-95,4% Aktivsubstanz in Wasser; INCI-Bezeichnung: Aminomethyl Propanol) (Dow Chemical)
- Fixate Plus: Copolymer aus PEG-25 C10-30 Alkylethermethacrylat, PEG/PPG-5/5 Allylether und einem oder mehreren Monomern, ausgewählt aus Acrylsäure, Methacrylsäure und deren einfachen Estern (ca. 30 %ig) INCI-Bezeichnung: Polyacrylate-14 (Lubrizol)

Alle Zusammensetzungen hatten den pH-Wert 7,2.

Die Viskostät wurde bei 20°C mittels eines Haake Rotationsviskosimeters, Spindel MVII, Schergeschwindigkeit 8 upm, bestimmt:

| | **V1** | **E** | **V2** |
|---|---|---|---|
| Viskosität (mPas) | 981,7 | 4555 | 47,05 |

Die Ergebnisse zeigen, daß nur das erfindungsgemäße Mitte E eine ausreichend hohe Viskosität aufweist, die Zusammensetzungen V1 und V2, bei denen jeweils eines der Polymere (A) oder (B) fehlt, besitzen zu geringe Viskositäten.

Versucht man, die Mittel V1 bzw. V2 in ihrer Viskosität zu erhöhen, indem die jeweiligen Polymermengen erhöht werden, so erhält man klebrige Mittel, die sich schlecht auftragen lassen und ein äußerst unangenehmes Hautgefühl besitzen.

## Patentansprüche

1. Kosmetische oder dermatologisch-topische Zusammensetzung, umfassend in einem wässrigen oder wässrig-alkoholischen Träger - jeweils bezogen auf das Gewicht der anwendungsbereiten Zusammensetzung -
• (A) 0,01 bis 5 Gew.-% mindestens eines ersten Copolymers, ausgewählt aus Copolymeren von
o (a1) mindestens einem sauren Vinylmonomer, ausgewählt aus Acrylsäure und Methacrylsäure oder einem Salz davon,
o (a2) mindestens einem hydrophoben nichtionischen Vinylmonomer, ausgewählt aus Acrylsäureestern und Methacrylsäureestern,
o (a3) mindestens einem ersten assoziativen Monomer und
o (a4) mindestens einem Monomer, das ausgewählt ist aus der Gruppe bestehend aus einem zweiten assoziativen Monomer, das sich von dem ersten assoziativen Monomer unterscheidet, einem semihydrophoben Monomer und einer Kombination davon; wobei die assoziativen Monomere (i) eine ethylenisch ungesättigte Endgruppe, (ii) einen hydrophilen Mittelabschnitt und (iii) eine hydrophobe oder semihydrophobe Endgruppe aufweisen;
• (B) 0,01 bis 2,5 Gew.-% mindestens eines zweiten Homo- und/oder Copolymers, ausgewählt aus ganz oder teilweise neutralisierten Homo- und/oder Copolymeren der Acrylsäure und/oder Methacrylsäure,
• (C) 1 bis 35 Gew.-% eines oder mehrerer Tenside.

2. Zusammensetzung nach Anspruch 1, **dadurch gekennzeichnet, daß** die nichtionischen Vinylmonomere (a2) ausgewählt sind aus Acrylsäure-C₁₋₄-alkylestern und Methacrylsäure-C₁₋₄-alkylestern und wobei das Monomer (a4) ein assoziatives Monomer ist, das sich von (a3) in den hydrophoben Endgruppen unterscheidet, die unabhängig voneinander aus C₈- bis C₄₀-Kohlenwasserstoffendgruppen ausgewählt sind.

3. Zusammensetzung nach einem der Ansprüche 1 oder 2, **dadurch gekennzeichnet, daß** die Monomermischung als hydrophobes nichtionisches Vinylmonomer (a2) PEG-25 C10-30 Alkylether Methacrylat umfaßt.

4. Zusammensetzung nach einem der Ansprüche 1 bis 3, **dadurch gekennzeichnet, daß** die Monomermischung mindestens ein semihydrophobes Monomer (a4) umfasst, das ausgewählt ist aus Monomeren mit einer der folgenden Formeln
R¹CH=CR¹-A-(CH₂)ₚ-(O)ᵣ-(R³O)ᵥ-R⁴
oder
D-A-(CH₂)ₚ-(O)ᵣ(R³O)ᵥ-R⁴
wobei in jeder der Formeln jedes R¹ unabhängig H, C₁-C₃₀-Alkyl, -C(O)OH oder -C(O)OR² ist; R² C₁-C₃₀-Alkyl ist; A -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- oder -CH₂CH₂NHC(O)- ist; Ar ein zweiwertiges Aryl ist; E H oder Methyl ist; z 0 oder 1 ist; p eine ganze Zahl im Bereich von 0 bis etwa 30 ist und r 0 oder 1 ist, mit der Maßgabe, dass, wenn p 0 ist, r 0 ist, und wenn p im Bereich von 1 bis etwa 30 liegt, r 1 ist; (R³O)ᵥ ein Polyoxyalkylen ist, welches ein Homopolymer, ein statistisches Copolymer oder ein Blockcopolymer von C₂-C₄-Oxyalkyleneinheiten ist, worin R³ C₂H₄, C₃H₆, C₄H₈ ist und v eine ganze Zahl im Bereich von etwa 5 bis etwa 250 ist, R⁴ H oder C₁-C₄-Alkyl ist; und D ein ungesättigtes C₈-C₃₀-Alkyl oder ein carboxysubstituiertes ungesättigtes C₈-C₃₀-Alkyl ist.

5. Zusammensetzung nach Anspruch 4, **dadurch gekennzeichnet, daß** die Monomermischung mindestens ein semihydrophobes Monomer (a4) umfasst, das ausgewählt ist aus Monomeren mit einer der folgenden Formeln
CH₂=CH-O-(CH₂)ₐ-O-(C₃H₆O)_{b}-(C₂H₄O)_{c}-H
oder
CH₂=CH-CH₂-O-(C₃H₆O)_{d}-(C₂H₄O)ₑ-H;
worin a 2, 3 oder 4 ist; b eine ganze Zahl im Bereich von 1 bis etwa 10 ist; c eine ganze Zahl im Bereich von etwa 5 bis etwa 50 ist; d eine ganze Zahl im Bereich von 1 bis etwa 10 ist; und e eine ganze Zahl im Bereich von etwa 5 bis etwa 50 ist.

6. Zusammensetzung nach einem der Ansprüche 1 bis 5, **dadurch gekennzeichnet, daß** sie - bezogen auf ihr Gewicht - 0,02 bis 4 Gew.-%, vorzugsweise 0,05 bis 3 Gew.-%, weiter bevorzugt 0,1 bis 2,5 Gew.-%, noch weiter bevorzugt 0,25 bis 1,5 Gew.-% und insbesondere 0,5 bis 0,95 Gew.-% Copolymer(e) (A) enthält.

7. Zusammensetzung nach einem der Ansprüche 1 bis 6, **dadurch gekennzeichnet, daß** sie als zweites Homo- und/oder Copolymer, ausgewählt aus ganz oder teilweise neutralisierten Homo- und/oder Copolymeren der Acrylsäure und/oder Methacrylsäure, ganz oder teilweise neutralisierte Homo- und/oder Copolymere der Acrylsäure mit Molmassen von 200 bis 10.000 kDa, vorzugsweise von 250 bis 7500 kDa und insbesondere von 500 bis 5000 kDa enthält.

8. Zusammensetzung nach einem der Ansprüche 1 bis 7, **dadurch gekennzeichnet, daß** sie - bezogen auf ihr Gewicht - 0,02 bis 2,0 Gew.-%, vorzugsweise 0,05 bis 1,5 Gew.-%, weiter bevorzugt 0,1 bis 1,25 Gew.-%, noch weiter bevorzugt 0,25 bis 1,0 Gew.-% und insbesondere 0,5 bis 0,95 Gew.-% ganz oder teilweise neutralisierte Homo- und/oder Copolymere der Acrylsäure und/oder Methacrylsäure enthält.

9. Zusammensetzung nach einem der Ansprüche 1 bis 8, **dadurch gekennzeichnet, daß** sie als zweites Homo- und/oder Copolymer, ausgewählt aus ganz oder teilweise neutralisierten Homo- und/oder Copolymeren der Acrylsäure und/oder Methacrylsäure ein oder mehrere Copolymer(e) aus der Gruppe der Polymere mit den INCI-Bezeichnungen
• Acrylamide/Sodium Acrylate Copolymer und/oder
• Acrylates/Aminoacrylates/C10-30 Alkyl PEG-20 Itaconate Copolymer und/oder
• Acrylates/C10-30 Alkyl Acrylate Crosspolymer und/oder
• Acrylates/Stearyl Methacrylate Copolymer und/oder
• Acrylates/Vinyl Isodecanoate Crosspolymer und/oder
• Allyl Methacrylates Crosspolymer und/oder
• Ammonium Polyacrylate und/oder
• Calcium Potassium Carbomer und/oder
• Corn Starch/Acrylamide/Sodium Acrylate Copolymer und/oder
• Potassium Carbomer und/oder
• Potassium Polyacrylate und/oder
• Sodium Acrylate/Acryloyldimethyl Taurate Copolymer und/oder
• Sodium Acrylate/Acryloyldimethyltaurate/Dimethylacrylamide Crosspolymer und/oder
• Sodium Acrylate/Acryloyldimethyltaurate/Acrylamide Colymer und/oder
• Sodium Acrylates/Vinyl Isodecanoate Crosspolymer und/oder
• Sodium Acrylate/Vinyl Alcohol Copolymer und/oder
• Sodium Polyacrylate Starch und/oder
• Sodium Polymethacrylate
enthält.

10. Verwendung von Mischungen aus
• (A) mindestens einem ersten Copolymer, ausgewählt aus Copolymeren von
o (a1) mindestens einem sauren Vinylmonomer, ausgewählt aus Acrylsäure und Methacrylsäure oder einem Salz davon,
o (a2) mindestens einem hydrophoben nichtionischen Vinylmonomer, ausgewählt aus Acrylsäureestern und Methacrylsäureestern,
o (a3) mindestens einem ersten assoziativen Monomer und
o (a4) mindestens einem Monomer, das ausgewählt ist aus der Gruppe bestehend aus einem zweiten assoziativen Monomer, das sich von dem ersten assoziativen Monomer unterscheidet, einem semihydrophoben Monomer und einer Kombination davon;
wobei die assoziativen Monomere (i) eine ethylenisch ungesättigte Endgruppe, (ii) einen hydrophilen Mittelabschnitt und (iii) eine hydrophobe oder semihydrophobe Endgruppe aufweisen;
• (B) mindestens einem zweiten Homo- und/oder Copolymer, ausgewählt aus ganz oder teilweise neutralisierten Homo- und/oder Copolymeren der Acrylsäure und/oder Methacrylsäure,
• (C) mindestens eines Tensids
zur Verbesserung des Hautgefühls kosmetischer oder dermatologisch-topischer Zusammensetzungen.
